# FASCICULE DE BREVET EUROPEEN

(11) **EP 2 616 536 B1**
(45) Date de publication et mention de la délivrance du brevet: **15.11.2017**
(21) Numéro de dépôt: 11773088.7
(22) Date de dépôt: 15.09.2011
(51) Int. Cl.: C12N 1/12, C12N 13/00, C12P 7/64

(54) **PROCÉDÉ DE CULTURE D'ALGUES UNICELLULAIRES MIXOTROPHES EN PRÉSENCE D'UN APPORT LUMINEUX DISCONTINU SOUS FORME DE FLASHS**
VERFAHREN ZUR KULTIVIERUNG VON MIXOTROPHEN EINZELLIGEN ALGEN IN GEGENWART EINER UNTERBROCHENEN LICHTVERSORGUNG IN FORM VON BLITZEN
METHOD FOR CULTURING MIXOTROPHIC SINGLE-CELL ALGAE IN THE PRESENCE OF A DISCONTINUOUS PROVISION OF LIGHT IN THE FORM OF FLASHES

(30) Priorité: 15.09.2010 FR 1057380
(43) Date de publication de la demande: 24.07.2013
(73) Titulaire: Fermentalg, 33500 Libourne (FR)
(72) Inventeur: CALLEJA, Pierre, F-33000 Bordeaux (FR)
(74) Mandataire: Be IP
(86) Numéro de dépôt international: PCT/FR2011/052114
(87) Numéro de publication internationale: WO 2012/035262

(56) Documents cités:
- WO-A1-96/21723
- WO-A1-2006/020177
- WO-A1-2009/134114
- OGBONNA JAMES C ET AL: "Light requirement and photosynthetic cell cultivation: Development of processes for efficient light utilization in photobioreactors", JOURNAL OF APPLIED PHYCOLOGY, vol. 12, no. 3-5, octobre 2000 (2000-10), pages 207-218, XP002632291, ISSN: 0921-8971
- WU X ET AL: "A model integrating fluid dynamics in photosynthesis and photoinhibition processes", CHEMICAL ENGINEERING SCIENCE 20010618 ELSEVIER LTD GB, vol. 56, no. 11, 18 juin 2001 (2001-06-18) , pages 3527-3538, XP002632292, DOI: DOI:10.1016/S0009-2509(01)00048-3
- SAVIDGE G: "GROWTH AND PHOTOSYNTHETIC RATES OF PHAEODACTYLUM-TRICORNUTUM IN A CYCLICAL LIGHT FIELD", JOURNAL OF EXPERIMENTAL MARINE BIOLOGY AND ECOLOGY, vol. 100, no. 1-3, 1986, pages 147-164, XP002632293, ISSN: 0022-0981
- KATSUDA T ET AL: "Effect of light intensity and frequency of flashing light from blue light emitting diodes on astaxanthin production by Haematococcus pluvialis", JOURNAL OF BIOSCIENCE AND BIOENGINEERING, ELSEVIER, AMSTERDAM, NL, vol. 105, no. 3, 1 March 2008 (2008-03-01) , pages 216-220, XP022590035, ISSN: 1389-1723, DOI: 10.1263/JBB.105.216 [retrieved on 2008-03-01]

## Description

La présente invention se rapporte à un nouveau procédé de culture d'algues unicellulaires (microalgues) en condition mixotrophe, permettant, notamment l'enrichissement du contenu lipidique de ces algues.

Ce procédé repose sur un apport de lumière dans le milieu de culture variable ou discontinu au cours du temps, lequel apport induit une augmentation du rendement des cultures et du contenu lipidique des algues.

### Préambule

Les algues unicellulaires font l'objet actuellement de nombreux projets industriels en vue de leur utilisation directe, en tant que complément alimentaire, ou indirecte en tant que matière première pour la chimie verte.

Les lipides issus de ces microalgues sont particulièrement recherchés car ils présentent d'excellentes qualités nutritionnelles. En particulier, ils contiennent des acides gras polyinsaturés à longues chaînes (PUFA) essentiels, comme l'EPA et le DHA, lesquels sont utilisés, notamment, dans la formulation des substituts de lait maternels. En aquaculture, les microalgues servent de substitut aux huiles et farines de poisson.

Du fait qu'en conditions favorables les microalgues peuvent accumuler jusqu'à 80 % de leur poids sec en acides gras, celles-ci offrent une alternative crédible à la culture des plantes terrestres oléagineuses pour la production de biocarburants [Li, Y et al., 2008 Biotechnol. Prog., 24: 815-820].

Les algues unicellulaires se présentent comme des microorganismes photosynthétiques au caractère autotrophe, c'est-à-dire qu'elles ont l'aptitude de croître de manière autonome par photosynthèse.

La plupart des espèces d'algues unicellulaires rencontrées dans l'eau douce ou les océans sont strictement autotrophes, c'est-à-dire qu'elles ne peuvent croître autrement que par photosynthèse. Pour celles-ci, la présence dans leur milieu de substrats carbonés ou de matières organiques ne leur est pas favorable et tend même à inhiber leur croissance.

Cependant, un certain nombre d'espèces d'algues unicellulaires, de familles et d'origine très diverses, s'avèrent ne pas être strictement autotrophes. Certaines d'entre-elles, dites hétérotrophes, sont capables de se développer en l'absence totale de lumière, par fermentation, c'est-à-dire en exploitant la matière organique.

D'autres espèces d'algues, pour lesquelles la photosynthèse reste indispensable à leur développement, sont capables de tirer parti, à la fois, de la photosynthèse et de la matière organique présente dans leur milieu. Ces espèces intermédiaires, dites mixotrophes, peuvent être cultivées à la fois en présence de lumière et de matière organique.

Cette particularité des algues dites « mixotrophes » semble être liée à leur métabolisme, qui leur permet d'opérer simultanément photosynthèse et fermentation. Les deux types de métabolisme coexistent avec un effet global positif sur la croissance des algues [Yang C. et al. (2000) Biochemical Engineering Journal 6 :87-102]*.*

A l'heure actuelle, la classification des algues se fonde encore largement sur des critères morphologiques et sur la nature des pigments photosynthétiques qu'elles contiennent. Cette classification renseigne peu sur le caractère autotrophe, hétérotrophe ou mixotrophe, alors que celles-ci recouvrent une très grande diversité d'espèces et de formes [Dubinsky et al. 2010, Hydrobiologia, 639:153-171].

De ce fait, une souche est considérée comme étant mixotrophe dès lors que, de manière expérimentale, il peut être prouvé qu'elle a la capacité de croître par photosynthèse dans un milieu minéral, dans lequel est ajouté un substrat carboné tel que du glucose, de l'acétate ou du glycérol. Si cette supplémentation en substrat carboné ne donne pas lieu à une inhibition de croissance durant la phase éclairée, alors la souche peut être considérée comme étant mixotrophe.

Si, pour certaines espèces d'algues, le fait de pouvoir croître en conditions mixotrophes permet théoriquement d'atteindre, en culture, un meilleur rendement en ce qui concerne la biomasse cellulaire et la production de lipides [Ceron Garcia M.C. et al. 2000, Journal of Applied Phycology, 13 :239-248], un tel niveau de rendement n'est que rarement atteint.

En effet, la gestion du rapport quantité de lumière/quantité de substrat carboné à introduire dans le milieu de culture pour obtenir un rendement optimal, reste délicate à déterminer [Lee Y.K. et al. 2001, Journal of Applied Phycology, 13 :307-315], notamment pour ce qui est de produire, une quantité exploitable de lipides. Le brassage de culture décrit dans WO 2009/134114 ne permet pas de contrôler l'apport de lumière.

Il subsiste donc un besoin d'améliorer les procédés de culture actuels des microalgues mixotrophes pour obtenir des conditions de production assurant une biomasse importante et des concentrations élevées de lipides.

Des travaux menés sur différentes espèces de microalgues ont établi que l'intensité lumineuse avait une influence directe sur le rendement quantitatif et qualitatif des cultures effectuées en mode mixotrophe. Katsuda & al. [J. of Bioscience and Bioengineering, 2008, 105 - 3, 216-220] décrivent l'usage de flashs pour étudier la production d'astaxanthin par H. pluvialis.

Une intensité lumineuse égale à celle pratiquée en mode autotrophe est généralement trop forte pour pratiquer la culture des algues en mode mixotrophe. En raison de ce phénomène de photo-inhibition, il convient souvent de réduire l'intensité lumineuse pour obtenir de meilleurs rendements [Liang Y. et al., 2009, Biotechnol. Lett., 31 :1043-1049 ; Chojnacka, K. et al., 2004, Enzyme and Microbial Technology, 34 :461-465 ; Bouarab L. et al., 2004, Water Research, 38:2706-2712; Jeon Y.C. et al. 2006, Enzyme and Microbial Technology, 39 :490-495].

Du fait que les cultures d'algues, à l'échelle industrielle, s'effectuent souvent à ciel ouvert, c'est-à-dire avec un éclairement naturel, les expériences réalisées pour déterminer les conditions optimales de cultures en mode mixotrophe, sont généralement réalisées avec des phases d'éclairement continues. Dans certains cas, une phase éclairée alterne avec une phase obscure de façon quotidienne (photopériode), reproduisant ainsi le jour et la nuit. Toutefois, il n'est pas fait état, dans l'art antérieur, d'alternances répétées ou de variations significatives au cours du temps de l'intensité lumineuse apportée aux cultures.

Des expériences antérieures portant sur des cultures alternant des phases de culture en mode autotrophe (éclairé) et hétérotrophe (obscur), de plusieurs heures chacune, se sont montrées décevantes en termes d'optimisation des cultures, du fait de la nécessité pour les algues de s'adapter aux changements de conditions de culture. Il a été observé une période de latence au début de chaque cycle, liée au phénomène dit de photo-activation, participant à une baisse de rendement des cultures [Ogbonna J.C. et al., 1997, Journal ofApplied Phycology 9 :359-366].

C'est sans doute la raison pour laquelle les auteurs des différents travaux d'optimisation de la culture des algues en mode mixotrophe, n'ont pas jugé qu'une alternance de phases éclairées et obscures pouvait avoir un effet favorable sur le rendement des cultures.

De façon surprenante, l'inventeur a constaté, au contraire, qu'un apport de lumière discontinu n'était pas préjudiciable au rendement des cultures réalisées en mode mixotrophe. Il a notamment observé qu'en faisant varier l'intensité lumineuse, soit par l'alternance rapprochée de phases obscures et de phases éclairées (flashs), soit en faisant fluctuer l'intensité lumineuse au cours du temps, il pouvait agir positivement sur le rendement des cultures en termes de biomasse, et plus particulièrement, sur le contenu lipidique des cellules.

Sur la base de ces observations, l'inventeur a mis au point un procédé de culture d'algues mixotrophes permettant d'atteindre, d'une part, une biomasse plus élevée, et d'autre part, d'enrichir le contenu des algues en lipides. Ce procédé est fondé sur un apport lumineux variable ou discontinu au cours du temps.

Ce procédé, objet de la présente demande, semble pouvoir se généraliser à de très nombreuses espèces d'algues unicellulaires mixotrophes.

Les différents aspects et avantages de l'invention sont détaillés ci-après.
**Figure 1** **:** Graphique comparant la biomasse de cultures de Tetraselmis réalisées respectivement en mixotrophie avec un apport de lumière sous forme de flash selon l'invention (Δ) et en mode autrotrophe (◆) i.e. en lumière continue.
**Figure 2** **:** Graphique comparant le contenu lipidique des cellules de Tetraselmis cultivées respectivement en mixotrophie avec un apport de lumière sous forme de flash selon l'invention (X) et en mode autrotrophe (□).

### Description détaillée

La présente invention a ainsi pour objet un procédé de culture d'algues unicellulaires permettant d'augmenter leur biomasse et d'enrichir leur contenu lipidique.

Ce procédé permet également de sélectionner des souches de microalgues particulièrement adaptées à la production de lipides en mode mixotrophe.

Ce procédé est caractérisé en ce que le flux de lumière apporté aux algues en culture est discontinu par flashage dont l'intensité en micromoles de photons varie d'une amplitude égale ou supérieure à 10 µmol. m⁻². s⁻¹ à raison de plusieurs fois par heure, les périodes d'obscurité étant d'au moins la moitié du temps durant lequel les algues sont cultivées ledit flashage consistant en des phases successives d'éclairement d'une durée comprise entre 10 secondes et 2 minutes.

Contrairement aux idées reçues, il est apparu qu'un éclairement discontinu des cultures, notamment en mode mixotrophe, avait un impact favorable sur le développement des algues et permettait d'accroître la production de lipides par ces dernières.

Sans être lié par la théorie, l'inventeur estime qu'un apport discontinu ou variable de lumière a pour effet de provoquer un stress chez les algues favorable à la synthèse des lipides. En effet, il est fréquent, dans la nature, que les algues accumulent des réserves lipidiques pour résister aux contraintes de leur environnement.

Selon un aspect préféré de l'invention, les phases successives d'éclairement sont comprises entre 20 secondes et 1 minute.

Selon l'invention, on peut aussi procéder à un apport lumineux alliant des phases d'éclairement continues et discontinues.

Selon un aspect préféré de l'invention, l'intensité lumineuse varie entre les valeurs 0 et 20 µmol. m⁻². s⁻¹, de préférence entre 0 et 50 µmol. m⁻².s⁻¹.

L'apport de lumière dans les cultures peut être obtenu par des lampes réparties autour de la paroi externe des fermenteurs. Une horloge déclenche ces lampes pour des temps d'éclairement définis. Les fermenteurs se situent préférentiellement dans une enceinte à l'abri de la lumière du jour, dont on peut contrôler la température ambiante.

Le procédé selon l'invention s'applique plus particulièrement aux algues unicellulaires capables de croître en condition de mixotrophie.

Comme indiqué dans le préambule de la présente demande, une culture d'algues en mode mixotrophe se définit comme une culture réalisée en mode autotrophe dans un milieu de culture enrichi en substrats carbonés.

De préférence, lesdits substrats carbonés comprennent, ou consistent en, de l'acétate, du glucose, de la cellulose, de l'amidon, du lactose, du saccharose ou du glycérol.

Au sens de la présente invention, une espèce d'algue est considérée comme mixotrophe, dès lors qu'elle peut être cultivée à la lumière, dans un milieu minimum (par exemple MM ou f/2) dans lequel est ajouté un substrat carboné à raison, par exemple, d'une concentration en carbone, glycérol ou acétate, équivalente ou supérieure à 5 mM, sans observer d'inhibition de croissance, c'est-à-dire sans constater de perte de biomasse en poids sec par rapport à une culture effectuée dans un même milieu minimum dépourvu de substrat carboné (c'est-à-dire en mode autotrophe).

Aux fins de la présente invention, on utilisera, de préférence, des microalgues mixotrophes, dont au moins 25 %, de préférence au moins 50 % de l'énergie produite par celles-ci, provient de l'exploitation par l'algue dudit substrat carboné.

Le milieu de culture doit contenir une quantité de substrat carboné suffisante pour la fermentation, mais pas trop élevée pour éviter d'inhiber la croissance des algues. De préférence, le milieu de culture selon l'invention comprend une concentration en glucose disponible inférieure à 10 g/L, préférentiellement comprise entre 4 et 6 g/L.

Selon un aspect préféré de l'invention le substrat carboné comprend de l'acétate, de préférence de l'acétate de sodium, dont la concentration dans le milieu de culture est généralement comprise entre 5 mM et 50 mM, préférentiellement entre 15 et 25 mM.

Avantageusement, on sélectionnera les espèces d'algues mixotrophes prises parmi les classes suivantes : *Euglenophyceae, Prasinophyceae, Eustigmatophyceae, Bacillariophyceae, Prymnesiophyceae, Pinguiophyceae, Dinophyceae, Trebouxiophyceae, Bicosoecophyceae, Katable-phariophyceae, Chlorophyceae, Haptophyceae, Raphido-phyceae, Chysophyceae, Coscinodiscophyceae, Alveolata, Bangiophyceae,*

*Rhodophyceae,* en particulier des souches productrices de lipides, et de préférence, des souches produisant au moins 5% de lipides en mode autotrophe.

Les microalgues appartenant à la classe des *Prasinophyceae,* sont de préférence du genre *Tetraselmis. sp.*

Un procédé de culture selon l'invention est, par exemple, réalisé en mixotrophie en présence d'un apport de lumière sous forme de flashs de lumière, de préférence entre 20 et 30 flashs par heure, dont l'intensité est généralement comprise entre 10 et 50 µmol. m⁻². s⁻¹, de préférence entre 10 et 15 µmol. m⁻². s⁻¹.

Selon un exemple préféré de l'invention, 30 flashs de 30 secondes par heure d'une intensité d'environ 10 µmol. m⁻². s⁻¹ sont appliqués à un milieu de culture comprenant du glucose ou de l'acétate de sodium et du calcium.

Le milieu de culture peut contenir d'autres éléments, tels que du potassium, du magnésium, des micro-éléments et des vitamines.

Un mode réalisation préféré consiste à utiliser la technique de culture en Fed-Batch, qui permet de maintenir le substrat carboné à des concentrations non inhibitrices, tout en favorisant une augmentation de la biomasse.

Préférentiellement, le glucose utilisé dans le milieu de culture est du D-glucose ou dextrose, en particulier du dextrose provenant de la biotransformation de l'amidon, par exemple à partir de maïs, de blé ou de pomme de terre. Les hydrolysats de l'amidon sont constitués de molécules de petite taille, qui sont également facilement assimilables par les algues, permettant d'obtenir un meilleur développement de la biomasse.

Selon un autre aspect de l'invention, la croissance des souches dépend de la présence dans le milieu de culture, de calcium en forte concentration, à savoir plus de 80 mg/L de calcium, et préférentiellement entre 120 et 190 mg/L.

Selon un mode de réalisation particulièrement adapté, le milieu de culture comprend entre 3 et 10 g/L de glucose et 100 et 200 g/L de calcium.

Avantageusement, un tel milieu permet d'obtenir un temps de latence d'environ 18 heures, et un temps de génération compris entre 3 et 4 heures.

Le procédé de culture selon l'invention doit être réalisé à une température moyenne permettant une bonne croissance des algues, préférentiellement comprise entre 4 et 32°C.

Dans ces conditions, on observe une réduction du temps de culture à moins de 40 heures, les temps de latence et de génération étant très faibles.

En tant que telles, les algues obtenues selon le procédé de l'invention, peuvent être utilisées dans l'alimentation, notamment l'alimentation animale, car elles sont potentiellement riches en protéines et en acides gras polyinsaturés (ex : EPA et DHA).

Selon un aspect préféré de l'invention, le procédé de culture précédemment défini permet également la production de lipides.

Ce procédé de production de lipides est caractérisé en ce qu'il comprend une ou plusieurs des étapes suivantes :
i) on met en culture des algues unicellulaires selon le procédé précédemment décrit, et
ii) on récupère les algues unicellulaires cultivées à l'étape i), et
iii) on extrait les lipides du contenu intracellulaire des algues récupérées à l'étape ii).

Les lipides peuvent être extraits en lysant les cellules et fractionnés selon les techniques connues de l'homme du métier.

Les lipides ainsi obtenus sont utiles dans différentes applications, sous formes d'acides gras polyinsaturés, notamment en tant que suppléments alimentaires tels que des substituts d'huiles de poisson, ou sous forme de triglycérides, par exemple pour la production de biocarburants.

Les exemples qui suivent ont pour but d'illustrer l'invention sans lui conférer de limitation.

### Exemples

### I - Ciblage de souches de Tetraselmis sp. mixotrophes

### 1 - Souches:

Les souches du genre *Tetraselmis* (n=78) ont été commandées auprès des souchothèques internationales CCAP (Ecosse), SAG (Allemagne), CCMP (USA) et CSIRO (Australie).

### 2 - Milieux de culture:

Les microalgues d'eau douce sont cultivées en autotrophie en milieu minimum MM liquide [50 mL/L Solution Beijerink (NH₄Cl 8g/L, CaCl₂ 1g/L, MgSO₄ 2g/L), 1 mL/L Tampon Phosphate (K₂HPO₄ 106 g/L, KH₂PO₄ 53 g/L), 1 mL/L solution d'oligoéléments (BO₃H₃ 11.4 g/L, ZnSO₄ 7H₂O 22 g/L, MnCl₂ 4H₂O 5.06 g/L, FeSO₄ 7H₂O 4.99 g/L, CoCl₂ 6H₂O 1.61 g/L, CuSO₄ 5H₂O 1.57 g/L, Mo₇O₂₄(NH4)₆ 4H₂O 1.1 g/L, EDTA 50g/L), 2.42 g/L Trizma base, pH ajusté entre 7.2 et 7.4 par HCl, 1.2 mg/L vitamine B₁ et 0.01 mg /L vitamine B₁₂ (ajoutées extemporanément)] et en milieu MM solide (+1.5 % agar).

Dans un premier temps, les microalgues marines sont cultivées en autotrophie en eau de mer reconstituée ou milieu f/2 liquide [NaNO₃ 0.64 g/L, KCI 0.74 g/L, NaCl ou Tropic marin salt 26g/L, CaCl₂ 1 g/L, MgSO₄ 7H₂O 1.92 g/L, NaH₂PO₄ 2H₂O 50 mg/L, 1 mL/L solution d'oligoéléments (Na₂EDTA 2H₂O 4.36 g/L, FeCl₃ 6H₂O 5.82 g/L, MnCl₂ 4H₂O 2.46 g/L, ZnSO₄ 7H₂O 34.5 mg/L, CoCl₂ 6H₂O 12 mg/L, CuSO₄ 5H₅O 9.8 mg/L, Na₂MoO₄ 2H₂O 2.2 mg/L), pH ajusté entre 7.2 et 7.4 par HCl, 0.1 mg/L vitamine B₁, 0.6 mg/L vitamine B₈ et 0.6 mg/L vitamine B₁₂ (ajoutées extemporanément)] et en milieu f/2 solide (+ 1.2 % agar).

Les cultures de microalgues en mixotrophie et en hétérotrophie ont été réalisées sur milieu MM ou f/2 additionné respectivement des substrats carbonés suivants: acétate 5 mM, glucose 5 g/L, lactose 10 g/L, saccharose 10 g/L et glycérol 5 g/L.

### 3 - Criblage Haut-Débit de la croissance de souches en milieu liquide en microplaques 24 et 96 puits:

Le caractère hétérotrophe et mixotrophe des souches de *Tetraselmis* a été évalué par une mise en culture des souches de microalgues aussitôt réceptionnées en milieu MM (souche d'eau douce) ou f/2 (souche marine) en présence d'un substrat carboné en microplaques 24 puits (V=2mL) ou 96 puits (V=1 mL) scellées par un film perméable aux gaz. Un contrôle de croissance en autotrophie (MM ou f/2) a été systématiquement effectué pour servir de référence aux cultures en mixotrophie et hétérotrophie.

Les microplaques ont été placées en chambre d'incubation (SANYO MLR-351H) à 22°C, 60 % d'humidité et 10µE d'intensité lumineuse pour les cultures en autotrophie et mixotrophie et en chambre d'incubation (BINDER KB53) à 22°C, 60 % d'humidité et à l'obscurité (0 µE) pour les cultures en hétérotrophie.

La chambre d'incubation a été modifiée afin d'apporter un éclairage régulé sous forme de flashs de lumière de 10 µE à raison de 30 flashs de 30 secondes par heure.

### 4 - Criblage haut-débit par spectrofluorimétrie du contenu lipidique intracellulaire des souches de microalgues cultivées respectivement en mixotrophie et hétérotrophie:

Après 3 à 4 semaines de suivi de croissance en microplaques 24 ou 96 puits, le contenu lipidique intracellulaire des microalgues mixotrophes et hétérotrophes a été évalué par spectrofluorimétrie. Les lipides intracellulaires ont été marqués spécifiquement par un fluorochrome, le Nile Red.

En réponse à une excitation de fluorescence à 488 nm, les lipides neutres marqués au Nile Red émettent une fluorescence à 570 nm et les lipides polaires à 610-620 nm.

A cet effet, les cultures liquides de souches de microalgues réalisées en conditions mixotrophes et hétérotrophes sont transférées en microplaques PCR 96 puits (V= 100 à 200 µl de culture) et marquées avec 1-2 µl de Nile Red (0.1 mg/mL). Après 20 min d'incubation à l'obscurité, la microplaque PCR est placée dans un spectrofluorimètre (Varian) et un scan d'émission de fluorescence de 500 à 700 nm est réalisé.

L'effet des flashs de lumière (colonnes autotrophie et mixotrophie) et de substrats carbonés (colonnes mixotrophie et hétérotrophie) tels que le glucose (Glc 5g/L), l'acétate (Ac 1 g/L), le saccharose (Sac 10g/L), le lactose (Lac 10g/L) et le glycérol (Gly 5g/L) sur la croissance des 78 souches du genre *Tetraselmis* a été évalué par criblage en microplaques 96 puits sur milieu liquide MM ou f/2. Le suivi de croissance est réalisé durant 3 à 4 semaines de façon bi-hebdomadaire par observation macroscopique des cultures et microscopique à la binoculaire (objectifs 10X et 32X).

### 5- Résultats:

Sur les 78 souches testées du genre *Tetraselmis*, 22 ont présenté un caractère hétérotrophe et seulement 7 souches se sont avérées strictement autotrophes. On observe en effet que les 22 souches hétérotrophes présentent une croissance significative à 0 µE sur un des substrats testés, comparable voire supérieure à celle en autotrophie. Le substrat carboné préférentiel des souches du genre *Tetraselmis* semble être le glucose avec 17 souches mixotrophes sur 21. Les souches sont dites strictement autotrophes lorsque l'ajout d'un substrat carboné en présence ou en l'absence de lumière n'améliore pas la croissance cellulaire par rapport à la culture en autotrophie. L'apport de lumière (10 µE) et du substrat carboné ont permis, dans le cas présent, à 71 souches du genre *Tetraselmis* d'atteindre une concentration cellulaire comparable ou supérieure à celle du contrôle en autotrophie. La culture en mixotrophie constitue donc la meilleure condition pour cultiver le plus grand nombre de souches du genre *Tetraselmis.*

L'effet de la lumière sur l'accumulation de lipides intracellulaires a été évalué chez les 21 souches mixotrophes du genre *Tetraselmis* capables de croître en conditions de mixotrophie en éclairage continu et discontinu, en microplaques 96 puits, en conditions de culture en phase stationnaire. Pratiquement, un aliquote de culture (200 µl) de chacune des 21 souches de *Tetraselmis* hétérotrophes est transféré sur microplaque 96 puits, puis marqué au Nile Red (1 µg/mL). Le signal d'émission de fluorescence, reflétant l'accumulation de lipides intracellulaires (lipides neutres à 570 nm et lipides polaires à 620 nm), est collecté au moyen d'un spectrofluorimètre.

Pour les 21 souches testées, il est apparu un marquage plus intense au Nile Red des souches cultivées en présence de flashs, par rapport à celles cultivées en mode éclairé continu.

### II - Croissance des souches de Tetraselmis sp. mixotrophes en mode flash

### 1 - Culture des souches de Tetraselmis sp. en bioréacteur:

Deux souches de *Tetraselmis*, prises au hasard parmi les 21 souches mixotrophes sélectionnées au cours du criblage décrit dans la partie I ci-dessus, ont été cultivées en mode flash selon l'invention dans des fermenteurs (fed-batch). Parallèlement, les mêmes souches ont été cultivées en autotrophie avec un éclairement continu.

Les cultures ont été réalisées dans des fermenteurs (BioController ADI 1030) de 2L utiles avec automates dédiés et supervision par station informatique. Le système a été régulé en pH via l'ajout de base (solution d'hydroxyde de sodium à 1N) et/ou d'acide (solution d'acide sulfurique à 1N). La température de culture est fixée à 23°C. L'agitation a été réalisée grâce à 3 mobiles d'agitation placés sur l'arbre selon la configuration de Rushton (hélices tripales à pompage descendant). La vitesse d'agitation et le débit d'aération ont été régulés au minimum à 100 rpm et au maximum à 250 rpm avec Qₘᵢₙᵢ = 0,5 wm / Qₘₐₓ = 2 wm respectivement. Le bioréacteur est équipé d'un système luminaire externe entourant la cuve transparente. L'intensité de la lumière émise, ainsi que les cycles de lumière ont été programmés à partir d'une station informatique. Les réacteurs ont été inoculés avec une pré-culture réalisée sur table agitante (140 rpm) en enceinte thermostatée (22°C), éclairée en continue à 100 µE. Les pré-cultures et cultures en bioréacteurs ont été réalisées en milieu f/2 supplémenté avec 10mM en NaHCo3. Le substrat carboné, qui a été utilisé pour la culture en mixotrophie dans le bioréacteur est l'acétate de sodium à une concentration de 20 mM. A partir de 92 h de culture, des ajouts de milieu f/2 concentré ont été réalisés toutes les 24h pour atteindre une concentration finale de 0.5X. Pour les cultures en mixotrophie «flash», 5 mM d'acétate de sodium ont été rajoutés en plus du milieu concentré f/2.

### 2 - Apport de lumière sous forme de flashs:

L'apport de lumière sous forme de flash dans les bioréacteurs a été à réalisé à l'aide de lampes LED réparties autour de la paroi externe desdits fermenteurs. Une horloge a déclanché ces lampes LED pour des temps d'éclairement ou des pulses entre 8 et 50 µE. L'intensité lumineuse du système flash est égale à celle utilisée en mode continu dans les cultures témoins en autotrophie.

### 3 - Suivi de la biomasse:

La concentration en biomasse totale a été suivie par mesure de la masse sèche (filtration sur filtre GFC, Whatman, puis séchage à l'étuve sous vide, 65°C et -0,8 bar, pendant 24h minimum avant pesée).

### 4 - Quantification des lipides intracellulaires:

La quantification des lipides totaux a été effectuée en prélevant des échantillons de 10⁷ cellules/mL. La méthodes d'extraction des lipides est celle décrite par Bligh, E.G. et Dyer, W.J. [A rapid method of total lipid extraction and purification (1959) Can. J.Biochem. Physiol 37:911-917].

### 5 - Résultats :

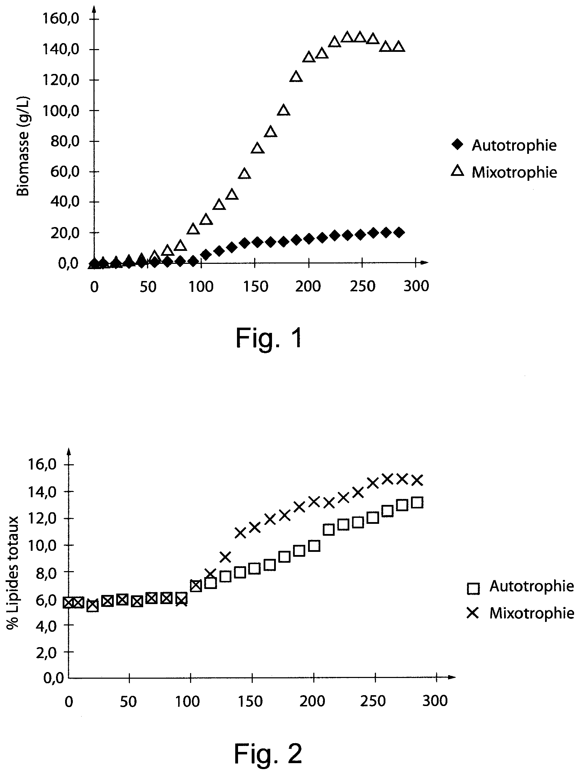

Les résultats obtenus pour les différentes cultures ont été reportés dans les graphiques des figures 1 et 2.

Le premier graphique indique un fort accroissement de la biomasse lorsque la culture est réalisée en mode flash (moyenne des différentes souches mixotrophes sélectionnées) par rapport à la culture des mêmes souches réalisées en mode autotrophe.

Le second graphique indique une accumulation de lipides dans les cellules cultivées en mode flash jusqu'à 30% supérieure à celle des cellules cultivées en mode autotrophe.

### 6 - Conclusion :

L'étude ci-dessus montre qu'un apport de lumière sous forme de flashs (30 flashs de 30 secondes par heure d'environ 10 µE) à des cultures de différentes microalgues du genre *Tetraselmis*, en plus des substrats carbonés, induit, chez toutes celles ayant un caractère mixotrophe, une accumulation significativement plus importante de lipides intracellulaires.

En termes de biomasse, les rendements ont été nettement plus importants en mode mixotrophe avec un apport de lumière sous forme de flash qu'en autotrophie. La concentration en microalgues obtenue est comprise entre 100 et 150 g/L (fig.1), ce qui est beaucoup plus élevé que les concentrations obtenues avec les cultures en lumière continue.

Par ailleurs, le procédé de culture d'algues unicellulaires selon l'invention permet de réduire le temps de culture des mêmes algues à moins de 40 heures, les temps de latence et de génération étant très faibles.

## Revendications

1. Procédé de culture d'algues unicellulaires cultivées en mode mixotrophe permettant d'augmenter leur biomasse et d'enrichir leur contenu lipidique, **caractérisé en ce que** lesdites algues sont cultivées en fermenteurs dans l'obscurité avec un apport de lumière discontinu par flashage dont l'intensité en micromoles de photons varie d'une amplitude égale ou supérieure à 10 µmol. m⁻². s⁻¹ à raison de plusieurs fois par heure, les périodes d'obscurité étant d'au moins la moitié du temps durant lequel les algues sont cultivées ledit flashage consistant en des phases successives d'éclairement d'une durée comprise entre 10 secondes et 2 minutes.

2. Procédé selon la revendication 1, **caractérisé en ce que** l'intensité lumineuse varie entre les valeurs 0 et 20 µmol. m⁻². s⁻¹.

3. Procédé selon l'une des revendications 1 ou 2, **caractérisé en ce que** l'intensité lumineuse varie entre 0 et 50 µmol. m⁻². s⁻¹.

4. Procédé selon l'une des revendications 1 à 3, **caractérisé en ce que** ledit flashage consiste en des phases successives d'éclairement d'une durée comprise entre 20 secondes et 1 minute.

5. Procédé selon la revendication 4, **caractérisé en ce que** ledit flashage consiste entre 20 et 30 flashs par heure.

6. Procédé selon l'une quelconque des revendications 1 à 5, **caractérisée en ce que** l'intensité des flashs est comprise entre 10 et 15 µmol. m⁻². s⁻¹.

7. Procédé selon l'une quelconque des revendications 1 à 6, **caractérisé en ce que** le milieu de culture est un milieu minimum supplémenté en en substrat carboné comprenant de l'acétate, du glucose, de la cellulose, de l'amidon, du lactose, du saccharose ou du glycérol.

8. Procédé selon la revendication 7, **caractérisé en ce que** le substrat carboné comprend majoritairement de l'acétate de sodium.

9. Procédé selon l'une quelconque des revendications 1 à 8, **caractérisé en ce que** les algues unicellulaires sont sélectionnées parmi les classes : *Euglenophyceae, Prasinophyceae, Eustigmatophyceae, Bacillariophyceae, Prymnesiophyceae, Pinguiophyceae, Dinophyceae, Trebouxiophyceae, Bicosoecophyceae, Katablephariophyceae, Chlorophyceae, Haptophyceae, Raphidophyceae, Chysophyceae, Coscinodiscophyceae, Alveolata, Bangiophyceae, Rhodophyceae.*

10. Procédé selon l'une quelconque des revendications 1 à 9, **caractérisé en ce que** les algues unicellulaires appartiennent à la classe des *Prasinophyceae,* de préférence au genre *Tetraselmis. sp.*

11. Procédé de production de lipides, **caractérisé en ce qu'**il comprend les étapes suivantes :
i) on met en culture des algues unicellulaires selon le procédé de culture de l'une quelconque des revendications 1 à 10, et
ii) on récupère les algues unicellulaires cultivées à l'étape i), et
iii) on extrait les lipides du contenu intracellulaire des algues récupérées à l'étape ii).

## Patentansprüche

1. Verfahren für die Kultur von einzelligen Algen, die im mixotrophen Modus kultiviert werden, das erlaubt, ihre Biomasse zu vergrößern und ihren Lipidinhalt anzureichern, **dadurch gekennzeichnet, dass** die Algen in Fermentern in Dunkelheit mit einer diskontinuierlichen Lichtzufuhr von Blitzen kultiviert werden, deren Intensität in Photonenmikromol in einer Amplitude von gleich oder über 10 µmol·M⁻²·s⁻¹ mehrmals pro Stunde schwankt, wobei die Perioden der Dunkelheit mindestens der Hälfte der Zeit entsprechen, in der die Algen kultiviert werden, wobei das Blitzen aus aufeinanderfolgenden Phasen der Erhellung einer Dauer zwischen 10 Sekunden und 2 Minuten inklusive besteht.

2. Verfahren nach Anspruch 1, **dadurch gekennzeichnet, dass** die Lichtintensität zwischen den Werten 0 und 20 µmol·M⁻²·s⁻¹ schwankt.

3. Verfahren nach einem der Ansprüche 1 oder 2, **dadurch gekennzeichnet, dass** die Lichtintensität zwischen den Werten 0 und 50 µmol·M⁻²·s⁻¹ schwankt.

4. Verfahren nach einem der Ansprüche 1 bis 3, **dadurch gekennzeichnet, dass** das Blitzen aus aufeinanderfolgenden Phasen der Erhellung einer Dauer zwischen 20 Sekunden und 1 Minute inklusive besteht.

5. Verfahren nach Anspruch 4, **dadurch gekennzeichnet, dass** das Blitzen aus 20 bis 30 Blitzen pro Stunde besteht.

6. Verfahren nach einem der Ansprüche 1 bis 5, **dadurch gekennzeichnet, dass** die Intensität der Blitze zwischen 10 und 15 µmol·M⁻²·s⁻¹.

7. Verfahren nach eine der Ansprüche 1 bis 6, **dadurch gekennzeichnet, dass** das Kulturmilieu ein Minimummilieu ist, supplementiert mit einem kohlenstoffhaltigen Substrat, umfassend Acetat, Glucose, Zellulose, Stärke, Laktose, Saccharose oder Glycerol.

8. Verfahren nach Anspruch 7, **dadurch gekennzeichnet, dass** das kohlenstoffhaltige Substrat mehrheitlich Natriumacetat umfasst.

9. Verfahren nach einem der Ansprüche 1 bis 8, **dadurch gekennzeichnet, dass** die einzelligen Algen aus den Klassen *Euglenophyceae, Prasinophyceae, Eustigmatophyceae, Bacillariophyceae, Prymnesiophyceae, Pinguiophyceae, Dinophyceae, Trebouxiophyceae, Bicosoecophyceae, Katablephariophyceae, Chlorophyceae, Haptophyceae, Raphidophyceae, Chysophyceae, Coscinodiscophyceae, Alveolata, Bangiophyceae, Rhodophyceae* ausgewählt sind.

10. Verfahren nach einem der Ansprüche 1 bis 9, **dadurch gekennzeichnet, dass** die einzelligen Algen zur Klasse der *Prasinophyceae,* vorzugsweise zur Gattung *Tetraselmis*·*sp*., gehören.

11. Verfahren der Lipidherstellung, **dadurch gekennzeichnet, dass** es die folgenden Schritte umfasst:
i) Kultivieren der einzelligen Algen gemäß dem Kulturverfahren nach einem der Ansprüche 1 bis 10, und
ii) Gewinnen der in Schritt i) kultivierten einzelligen Algen, und
iii) Extrahieren der Lipide des intrazellulären Inhalts der in Schritt ii) gewonnenen Algen.

## Claims

1. A method for culturing unicellular algae cultured in mixotrophic mode to increase their biomass and to enrich their lipid content, **characterized in that** said algae are cultured in fermenters in darkness with a supply of light that is discontinuous as flashes, the intensity of which in micromoles of photons varies with an amplitude greater than or equal to 10 µmol·m⁻²·s⁻¹ at a rate of several times per hour, wherein the periods of darkness are half of the time or more, during which the algae are cultured, said flashes consist of successive phases of illumination with a duration comprised between 10 seconds and 2 minutes.

2. The method according to claim 1, **characterized in that** the light intensity varies between the values 0 and 20 µmol·M⁻²·s⁻¹.

3. The method according to one of claims 1 or 2, **characterized in that** the light intensity varies between 0 and 50 µmol·M⁻²·s⁻¹.

4. The method according to one of claims 1 to 3, **characterized in that** said flashes consist of successive phases of illumination with a duration comprised between 20 seconds and 1 minute.

5. The method according to claim 4, **characterized in that** said flashes comprise between 20 and 30 flashes per hour.

6. The method according to any one of claims 1 to 5, **characterized in that** the intensity of the flashes is comprised between 10 and 15 µmol·M⁻²·s⁻¹.

7. The method according to any one of claims 1 to 6, **characterized in that** the culture medium is a minimum medium supplemented with carbon-containing substrate comprising acetate, glucose, cellulose, starch, lactose, sucrose or glycerol.

8. The method according to claim 7, **characterized in that** the carbon-containing substrate comprises predominantly sodium acetate.

9. The method according to any one of claims 1 to 8, **characterized in that** the unicellular algae are selected from the classes: *Euglenophyceae, Prasinophyceae, Eustigmatophyceae, Bacillariophyceae, Prymnesiophyceae, Pinguiophyceae, Dinophyceae, Trebouxiophyceae, Bicosoecophyceae, Katablephariophyceae, Chlorophyceae, Haptophyceae, Raphidophyceae, Chysophyceae, Coscinodiscophyceae, Alveolata, Bangiophyceae, Rhodophyceae.*

10. The method according to any one of claims 1 to 9, **characterized in that** the unicellular algae belong to the *Prasinophyceae* class, preferably to the genus *Tetraselmis sp.*

11. A method for producing lipids, **characterized in that** it comprises the following steps:
i) culturing unicellular algae according to the culture method of any one claims 1 to 10, and
ii) recovering the unicellular algae cultured in step i), and
iii) extracting the lipids from the intracellular contents of the algae recovered in step ii).
